Europäisches Patentamt

European Patent Office

Office européen des brevets

(11) Numéro de publication: **0 174 247 B1**

(12) **FASCICULE DE BREVET EUROPEEN**

(45) Date de publication de fascicule du brevet:
05.06.91

(51) Int. Cl.⁵ **G01N 33/548**

(21) Numéro de dépôt: **85401654.0**

(22) Date de dépôt: **19.08.85**

(54) **Bandelette d'analyse immunologique et procédé pour sa fabrication.**

(30) Priorité: **23.08.84 FR 8413114**

(43) Date de publication de la demande:
**12.03.86 Bulletin 86/11**

(45) Mention de la délivrance du brevet:
**05.06.91 Bulletin 91/23**

(84) Etats contractants désignés:
**AT BE CH DE GB IT LI LU NL SE**

(56) Documents cités:
**EP-A- 0 063 810     WO-A-83/01308
WO-A-84/03151     FR-A- 1 452 600
FR-A- 2 374 646     US-A- 3 941 876**

**CHEMICAL ABSTRACTS, vol. 99, no. 25, 19
décembre 1983, page 503, no. 210861b, Columbus, Ohio, US; V. HOREJSI et al.:
"Nitrocellulose membrane as an antigen or
antibody carrier for screening hybridoma
cultures" & J. IMMUNOL. METHODS 1983,
62(3), 325-9**

**CHEMICAL ABSTRACTS, vol. 98, no. 11, 14
mars 1983, page 243, no. 85532w, Columbus,
Ohio, US; I.C. SHEKARCHI et al.: "Microsticks
as solid-phase carriers for enzyme-linked**

**immunosorbent assays" & J. CLIN. MICRO-
BIOL. 1982, 16(6), 1012-18**

(73) Titulaire: **Guerin, Bernard
1, rue de l'Ecole de Mars
F-92200 Neuilly-sur-Seine(FR)**

Titulaire: **De Weck, Alain Ladislas
14, Grands Places
CH-1700 Fribourg(CH)**

(72) Inventeur: **Guerin, Bernard
1, rue de l'Ecole de Mars
F-92200 Neuilly-sur-Seine(FR)**
Inventeur: **De Weck, Alain Ladislas
14, Grands Places
CH-1700 Fribourg(CH)**

(74) Mandataire: **Lemoine, Michel et al
Cabinet Michel Lemoine et Bernasconi 13
Boulevard des Batignolles
F-75008 Paris(FR)**

## Description

La présente invention concerne, d'une façon générale, les analyses ou tests médicaux, notamment d'immunologie ou d'allergologie, et elle est plus particulièrement relative à des moyens destinés à être utilisés pour effectuer de tels analyses ou tests d'une façon commode, ainsi qu'à la fabrication de ces moyens.

On sait que ces analyses ou tests sont effectués en utilisant une phase solide sur laquelle sont fixés, par liaison stable, covalente ou non, des antigènes dont des allergènes ou des anticorps ; cette phase solide peut notamment être constituée par diverses membranes poreuses dont la plus connue est à base de nitrocellulose avec laquelle on met en contact des prélèvements sur des malades, puis les divers réactifs de révélation des réactions spécifiques antigènes-anticorps.

La nitrocellulose utilisée pour ces examens est une matière fragile, cassante facilement contaminable, qui implique des manipulations extrêmement délicates nécessitant notamment des conditions hygrométriques particulières.

On a, en conséquence, réalisé des feuilles composites formées d'un support sur lequel est fixée une feuille de nitrocellulose, comme décrit, par exemple, dans la demande de brevet européen n° 0063810 Ciba-Geigy AG, ces feuilles composites étant découpées en bandes suivant les besoins, pour chaque analyse.

Cependant, la nature fragile de la nitrocellulose n'a pas permis jusqu'à présent une fabrication en série automatisée et peu coûteuse de ces feuilles composites.

Par ailleurs, leur utilisation n'est pas extrêmement commode, notamment pour la lecture des résultats dans les appareils d'examen (réfractomètres, densimètres ou autres).

D'autre part, ces feuilles composites ont une sensibilité qui est loin d'être optimale et sont de plus difficiles à laver et à sécher.

L'invention a pour but de remédier à ces inconvénients en réalisant des moyens d'analyse du type ci-dessus sous forme individuelle pour chaque malade, d'une manipulation et d'un examen faciles aux instruments de mesure et présentant une sensibilité accrue, et en fournissant un procédé de fabrication industrielle de ces moyens.

Elle a pour objet à cet effet une bandelette d'analyse ou de test médical, notamment d'immunologie, du type précité, caractérisée en ce qu'elle comporte une pluralité de plages sucessives de membranes de nitrocellulose ou d'une autre membrane présentant les mêmes propriétés, fixées sur une étroite bandelette allongée de support en matière semi-rigide, chacune desdites plages comportant, d'une façon connue en soi, une zone imprégnée de réactif, la plage étant elle-même saturée de protéine, ladite bandelette présentant, au droit de chaque zone, une ou plusieurs perforations de grande surface par rapport à l'épaisseur de la membrane.

Les plages de membrane peuvent être reliées à la bandelette de support par collage mais de préférence elles sont soudées sur le support.

De préférence, les plages successives de membrane sont séparées les unes des autres par des espaces dépourvus de membrane.

La bandelette de support peut comporter des repères codés correspondant à chaque plage ou zone réactive. Elle peut également comporter, de façon avantageuse, des moyens d'indexation et/ou d'entraînement pour coopérer avec un appareil d'examen. Ces moyens d'indexation et/ou d'entraînement peuvent, par exemple, consister en un ou de préférence deux ou plusieurs trous ou perforations pratiqués de préférence dans une partie de la bandelette de support dépourvue de membrane, de préférence vers une extrémité de la bandelette.

L'invention a également pour objet un procédé de fabrication de ces moyens d'analyse ou de test, caractérisé en ce que l'on applique une feuille de membrane adsorbante de nitrocellulose ou d'un autre matériau présentant les mêmes propriétés sur une feuille de support en matière semi-rigide, préalablement perforée d'une pluralité de trous au droit des emplacements des zones de réactifs, que l'on fixe la feuille de membrane sur la feuille de support, que l'on applique les réactifs sur les zones de plages successives, et que l'on sature, d'une façon en soi connue, la membrane de protéine.

De préférence, les dimensions des feuilles de membrane et de support correspondent à une pluralité de bandelettes adjacentes et, dans ce cas, on découpe les feuilles superposées et fixées, à l'état encore humide après saturation de protéine.

La fixation de la feuille de membrane sur le support peut s'effectuer par collage, notamment avec des colles souples, du type colle caoutchouc, mais de préférence on réalise cette fixation par soudage à la chaleur ou aux ultrasons.

De façon particulièrement préférée, on peut réaliser cette fixation en enlevant la membrane par chauffage chaque fois entre deux plages successives, ce qui réalise simultanément le soudage des plages de membrane sur la feuille de support et la formation d'espaces dépourvus de membrane entre deux plages consécutives, le découpage final s'effectuant perpendiculairement aux lignes de soudage.

Suivant une autre caractéristique de l'invention, la préparation des feuilles composites est réalisée jusqu'à la dépose des réactifs en conservant, sur la face non encollée, un papier de protection de la membrane qui est seulement soulevé mécaniquement pendant la phase de brûlage des zones de séparation des plages réactives.

Suivant un mode de mise en oeuvre avantageux de l'invention, lesdits réactifs sont appliqués sous forme de traits rectilignes au moyen de styles mais, en variante, ils peuvent l'être sous forme de points grâce à des micropipettes.

D'autres avantages et caractéristiques de l'invention apparaîtront au cours de la description qui va suivre faite en se référant au dessin annexé donné uniquement à titre d'exemple et dans lequel :

la figure 1 est une vue de dessus d'une bandelette d'analyse immunologique suivant l'invention ;

la figure 2 est une vue en coupe longitudinale de cette bandelette ;

la figure 3 est une vue de dessus des feuilles de nitrocellulose et de support avant l'application des réactifs.

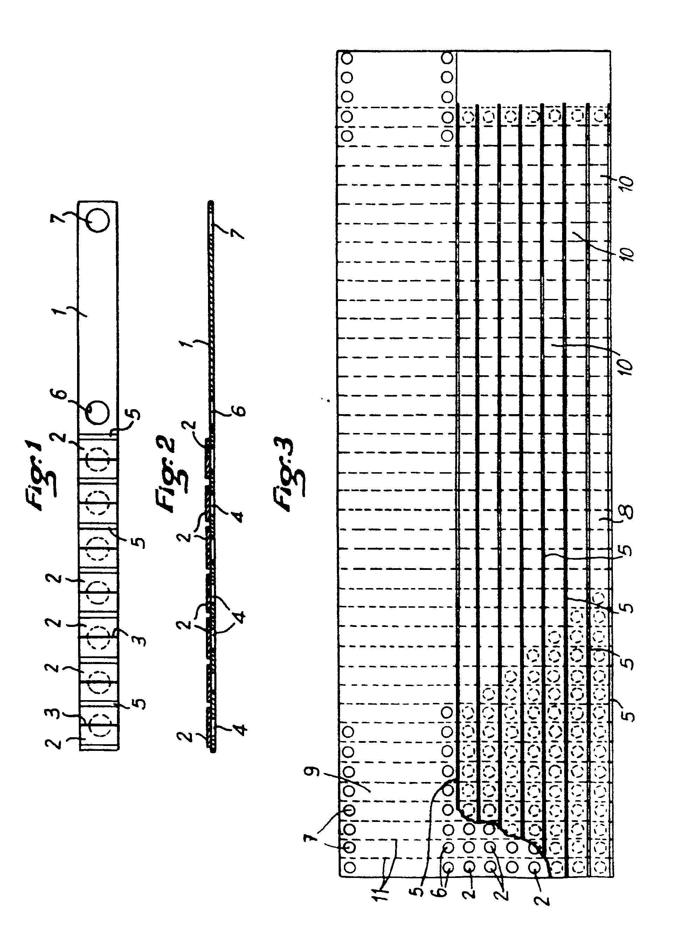

En se référant à la figure 1 du dessin, une bandelette individuelle d'analyse immunologique suivant l'invention comprend une lame ou bandelette de support 1 sur laquelle sont fixées à intervalles égaux des plages sous forme de plaquettes 2 de nitrocellulose.

Chaque plaquette 2 comporte une zone rectiligne ou ponctuelle 3 imprégnée d'un réactif tel qu'un allergène ou plus généralement un antigène ou un anticorps.

L' ensemble de chaque plaquette a, d'autre part, été imprégné et saturé de protéine, par exemple une albumine.

La lame 1 de support de chaque bandelette comporte au droit de chaque plage 2 une perforation 4 ayant un grand diamètre par rapport à l'épaisseur de membrane formant la plage et se projetant donc sur une grande partie de la zone rectiligne de réactif 3.

Dans cette forme de réalisation, chaque plage ou plaquette 2 est séparée de la plaquette adjacente par un espace 5 dont la largeur représentée est assez faible, mais qui pourrait être plus importante, et provenant, comme on le verra, de l'opération de soudage.

A l'extrémité de droite, la bandelette présente une portion de support 1 assez longue, dépourvue de nitrocellulose et munie de deux perforations écartées 6, 7 servant pour la fabrication, l'indexage et/ou l'avancement de la bandelette dans un appareil de lecture.

Lorsqu'on désire effectuer une analyse ou un test d' immunologie, on dépose sur chaque zone 3 de réactif une quantité appropriée d'un prélèvement effectué sur un malade puis de solution de lavage et de marquage, et on fait passer la bandelette dans un appareil de mesure tel qu'un réfractomètre, un densitomètre ou autre en faisant passer successivement chaque plaquette 2 dans les appareils d'examen à l'aide d'un mécanisme d'avance pas à pas d'un type quelconque approprié connu dans la technique (qui ne fait pas partie de l'invention).

La lame 1 est avantageusement constituée d'une matière semi-rigide telle que, par exemple, du chlorure de polyvinyle (PVC). Son épaisseur est de préférence de l'ordre de 200 a 800$\mu$.

La manipulation de ces bandelettes d'analyse est facile en raison de la nature non cassante du support tandis que la nitrocellulose est extrêmement fragile. En outre, le traitement des bandelettes par les différents réactifs est facile ainsi que la lecture des résultats des réactifs dans les appareils utilisés dans cette technique.

Les opérations de lavage et de séchage de la bandelette s'effectuent dans les meilleures conditions sur les deux faces de chaque zone 3 et on obtient une grande sensibilité de détection.

La séparation des plaquettes 2 par des parties dépourvues de nitrocellulose facilite l'application de la substance à examiner et évite des erreurs. En outre, des réactifs complémentaires peuvent être associés à la ou aux substances à examiner pour un même malade.

On se réfère maintenant à la figure 3 à l'aide de laquelle va être détaillé le procédé de fabrication des bandelettes selon l'invention.

On utilise initialement une feuille 8 de membrane nitrocellulose sur laquelle est appliquée une feuille de protection en un papier mince. L'épaisseur de la feuille 8 est de 100 à 200$\mu$ .

La face découverte de la feuille 8 de nitrocellulose est appliquée sur une feuille de support 9 en un matériau approprié semi-rigide tel que du chlorure de polyvinyle, la feuille de support 9 étant préalablement perforée par estampage d'une pluralité de trous 4, 6, 7 disposés en rangées longitudinales.

Ces trous 4, 6, 7 sont effectués par une presse et une matrice de poinçons. La face de la feuille de support 9 destinée à recevoir la feuille de membrane 8 est celle dépourvue des ébarbures de poinçonnage.

On peut facultativement enduire, par exemple à l'aide d'un rouleau, la face de la feuille 8 destinée à être appliquée contre le support 9, d'une colle, par exemple d'une colle souple du type solution de caoutchouc. La feuille 8 est saisie, du côté de la feuille de protection de papier qui la recouvre, à l'aide d'un plateau formant ventouse de transport puis est appliquée contre la feuille 9, le plateau formant presse. On obtient ainsi une excellente planéité de la membrane 8 sur la feuille de support 9.

Après avoir soulevé la feuille de papier de protection, on applique sur l'ensemble soit une rangée d'organes chauffants d'environ 1 mm de largeur chacun et on fait défiler l'ensemble de façon que les organes chauffants décrivent des trajectoires rectilignes formant des rainures 5 par élimination de la nitrocellulose sous-jacente, soit une pression avec un plateau composé de lignes chauffées et de zones réfrigérées, cette opération provoquant simultanément le soudage des bandes 10 de nitrocellulose délimitées entre les rainures 5, et ceci au niveau des bords desdites bandes 10.

On effectue ensuite le dépôt des réactifs sur les zones de test soit sous forme d'une microgoutte, soit sous forme d'un trait régulier 3 d'une largeur adaptée à la fente de l'appareil de lecture. Cette charge nécessite un dispositif d'avance à vitesse constante de l'ensemble des feuilles 8 et 9 et une batterie de micro-pipettes ou de styles de hauteur préréglable avec une grande précision à partir de la surface de la membrane de nitrocellulose. On comprend que chaque bande 10 reçoit un seul et même réactif.

Après ce traitement, l'ensemble est imprégné par exemple par trempage dans une solution d'albumine ou de caséine, plus économique et tout aussi efficace, qui pénètre la feuille 8 de nitrocellulose par sa face libre et à travers les trous 2 de la feuille de support. Après égouttage et séchage partiel, l'ensemble encore humide est découpé transversalement en bandelettes, par exemple au moyen d'un massicot comme indiqué par les traits interrompus 11, formant ainsi des bandelettes telles que représentées à la figure 1, présentant chacune des plages alternées 5 de PVC et des plaquettes 2 de nitrocellulose présentant chacune une zone 3 imprégnée de réactif.

Le massicot peut être muni d'ergots correspondant aux trous 6 et 7 pour réunir automatiquement toutes les bandelettes issues d'un même ensemble de feuilles 8 et 9 en les empilant.

D'une façon avantageuse, la feuille de support 1 en PVC ou polystyrène est pourvue de repères codés tels que Y dans des positions prédéterminées de façon à correspondre à chaque plaquette.

On effectue ensuite le séchage final jusqu'au dégré d'hygrométrie convenable, puis un contrôle d'activité, puis éventuellement l'impression des données d'étalonnage.

Les bandelettes peuvent ensuite être conditionnées en tubes ou sous forme individuelle formant, après ouverture, cuvette d'incubation pour les essais et tests ultérieurs.

**Revendications**

1. Bandelette d'analyse ou de test médical, notamment d'immunologie ou d'allergologie, du type comportant une membrane de nitrocellulose, fixée sur un support, caractérisée en ce qu'elle comporte :
   - une étroite bande allongée (1) réalisée en une matière semi-rigide formant support,
   - une pluralité de plages (2) successives de nitrocellulose fixées sur ledit support, chacune desdites plages comportant une zone (3) imprégnée d'un réactif, la plage étant elle-même imprégnée de protéine,
   - une pluralité de larges perforations (4) pratiquées dans ledit support au droit de chaque zone réactive.

2. Bandelette selon la revendication 1, caractérisée en ce que les plages de membrane (2) sont soudées sur le support.

3. Bandelette selon l'une quelconque des revendications 1 et 2, caractérisée en ce que les plages successives (2) sont séparées les une des autres par des espaces (5) dépourvus de membrane.

4. Bandelette selon l'une quelconque des revendications 1 à 3, caractérisée en ce que ladite feuille de support (1) est en chlorure de polyvinyle ou en polystyrène.

5. Bandelette selon l'une quelconque des revendications 1 à 4, caractérisée en ce que chaque zone (3) se présente sous forme d'un trait rectiligne ou d'une suite de points.

6. Bandelette selon l'une quelconque des revendications 1 à 5, caractérisée en ce que la feuille de support a une épaisseur comprise entre 200 et 800 microns.

7. Bandelette selon l'une quelconque des revendications 1 à 5, caractérisée en ce que la membrane de nitrocellulose a une épaisseur comprise entre 100 et 200 microns.

8. Bandelete selon l'une quelconque des revendications 1 à 7, caractérisée en ce qu'elle comporte des moyens d'indexation et/ou d'avancement (6,7).

9. Bandelete selon la revendication 8, caractérisée en ce qu' elle comporte, sur une extrémité du support (1) dépourvue de membrane, des trous (6,7).

10. Procédé de fabrication d'une bandelete d'analyse ou de test telle que définie dans l'une quelconque des revendications 1 à 9, caractérisé en ce que l'on applique une feuille (8) de membrane de nitrocellulose ou d'un autre matériau présentant les mêmes propriétés, sur une feuille de support (9) en une matière semi-rigide, préalablement perforée d'une pluralité de trous au droit des emplacements des zones de réactifs, en ce que l'on fixe la feuille de membrane (8) sur la feuille de support (9), en ce que l'on dépose les réactifs sur les zones de plages successives et en ce que l'on sature la membrane de nitrocellulose de protéine.

11. Procédé selon la revendication 10, caractérisé en ce qu à partir de feuilles (8, 9) destinées à former plusieurs bandelettes, on découpe l'ensemble des feuilles à l'état encore humide après saturation de protéine.

12. Procédé selon l'une quelconque des revendications 10 et 11, caractérisé en ce que l'on fixe la feuille de membrane (8) sur la feuille de support (9) par soudage à chaud ou aux ultrasons.

13. Procédé selon la revendication 12, caractérisé en ce que l'on réalise le soudage par chauffage en enlevant la membrane chaque fois entre deux plages (2) successives, formant ainsi des espaces (5) dépourvus de membrane entre des plages successives (2).

14. Procédé selon la revendication 13, caractérisé en ce que l'on recouvre la feuille de nitrocellulose (8) d'une mince feuille de protection et en ce que l'on soulève ladite feuille de protection pour découvrir la nitrocellulose avant le brûlage et le soudage des bandes sur le support (1).

15. Procédé selon l'une quelconque des revendications 10 à 14, caractérisé en ce que l'on colle la feuille de membrane (8) sur la feuille de support (9).

16. Procédé selon l'une quelconque des revendications 10 à 15, caractérisé en ce que l'on applique les réactifs sous forme de traits rectilignes à l'aide de styles ou de points à l'aide de micropipettes.

17. Procédé selon la revendication 11, caractérisé en ce que l'on découpe l'ensemble à l'état humide après l'imprégnation de protéine des bandelettes, transversalement aux bandes restantes, au moyen d'un massicot associé à des moyens d'empilage des bandelettes découpées.

18. Procédé selon la revendication 13, caractérisé en ce que l'on chauffe et en ce que l'on brûle lesdites bandes alternées de nitrocellulose au moyen d'une rangée transversale d'organes chauffants avançant sur la feuille (8) de membrane.

## Claims

1. A strip for analysis or medical testing, particularly in the field of immunology or allergology, of the type comprising a membrane of nitrocellulose bound to a support, characterised in that it includes :
   - a narrow elongated strip (1) made of a semi-rigid backing material,
   - a plurality of successive nitrocellulose areas (2) bound to said support, each of said areas including a reagent-impregnated zone (3), the area being itself protein-impregnated,
   - a plurality of perforations (4) with a large surface area made in said support opposite each reactive zone.

2. A strip according to claim 1, characterised in that the membrane areas (2) are bonded onto the support.

3. A strip according to any one of claims 1 and 2, characterised in that successive areas (2) are separated from each other by spaces (5) devoid of membrane.

4. A strip according to any one of claims 1 to 3, characterised in that said backing strip (1) consists of polyvinyl chloride or polystyrene.

5. A strip according to any one of claims 1 to 4, characterised in that each zone (3) takes the form of a linear streak or a succession of spots.

6. A strip according to any one of claims 1 to 5, characterised in that the backing strip has a thickness comprised between 200 and 800 microns.

7. A strip according to any one of claims 1 to 5, characterised in that the membrane of nitrocellulose has a thickness comprised between 100 and 200 microns.

8. A strip according to any one of claims 1 to 7, characterised in that it is provided with indexation and/or driving means (6,7).

9. A strip according to claim 8, characterised in that it includes holes (6,7) at one end of the support (1) devoid of membrane.

**10.** A process for making a strip for analysis or testing as defined in any one of claims 1 to 9, characterised in that a sheet (8) of nitrocellulose membrane or some other material with similar properties is applied to a backing sheet (9) of semi-rigid material which has previously been perforated with several holes opposite the location of the zones for the reagents, that the membrane sheet (8) is bound to the backing sheet (9), that the reagents are deposited on the successive zones of areas and that the membrane of nitrocellulose is saturated with protein.

**11.** Process according to claim 10, characterised in that an assembly of sheets intended for the production of several strips is cut while still moist after protein saturation.

**12.** Process according to any one of claims 10 and 11, characterised in that the membrane sheet (8) is bound to the backing sheet (9) by means of heat-treatment or ultrasound bonding.

**13.** Process according to claim 12, characterised in that the heat-bonding is carried out by removing the membrane between successive pairs of areas (2), so as to form spaces (5) without membrane between successive areas (2).

**14.** Process according to claim 13, characterised in that the nitrocellulose sheet (8) is covered by a thin protective sheet and that this protective sheet is removed to expose the nitrocellulose before the heat-treatment and bonding of the strips onto the support (1).

**15.** Process according to any one of claims 10 to 14, characterised in that the membrane sheet (8) is glued to the backing sheet (9).

**16.** Process according to any one of claims 10 to 15, characterised in that the reagents are applied in the form of linear streaks using styles or as spots by means of of micropipettes.

**17.** Process according to claim 11, characterised in that the assembly is cut whilst still moist following impregnation of the strips with protein, transversely to the remaining strips, using a trimmer combined with a mechanism for stacking the cut strips.

**18.** Process according to claim 13, characterised in that the alternating strips of nitrocellulose are heated and burned using a transversal row of heating elements progressing along the membrane sheet (8).

**Ansprüche**

**1.** Streifen für Analysen oder medizinische Teste, besonders in Immunologie oder Allergologie, bestehend aus einer auf einem Träger fixierten Nitrozellulosemembran, dadurch gekennzeichnet, dass er :
- einen schmalen und langen Streifen (1), der aus einem halbsteifen Trägermaterial hergestellt ist,
- mehrere aufeinanderfolgende Flächen (2) von Nitrozellulose, die an den Träger gebunden sind, wobei jede Fläche eine Stelle (3) enthält, die von einem Reagenz imprägniert ist, während die Stelle selbst mit Protein imprägniert ist,
- mehrere grosse Perforationen (4), die in den Träger gebohrt sind und gegenüber jeder reaktiven Stelle stehen, aufweist.

**2.** Streifen gemäss Anspruch 1, dadurch gekennzeichnet, dass die Membranflächen (2) auf dem Träger geschweisst sind.

**3.** Streifen gemäss einem der Ansprüche 1 und 2, dadurch gekennzeichnet, dass die aufeinanderfolgenden Stellen voneinander durch membranlose Zonen (5) getrennt sind.

**4.** Streifen gemäss einem der Ansprüche 1 bis 3, dadurch gekennzeichnet, dass die Trägerfolie (1) aus Polyvinylchlorid oder aus Polystyrol besteht.

**5.** Streifen gemäss einem der Ansprüche 1 bis 3, dadurch gekennzeichnet, dass jede reaktive Stelle (3) als eine gerade Linie oder als eine Nachfolge von Punkten ausgebildet ist.

**6.** Streifen gemäss einem der Ansprüche 1 bis 5, dadurch gekennzeichnet, dass die Dicke des Trägerstreifens zwischen 200 und 800 Microns liegt.

**7.** Streifen gemäss einem der Ansprüche 1 bis 5, dadurch gekennzeichnet, dass die Dicke der Nitrozellulosemembran zwischen 100 und 200 Microns liegt.

**8.** Streifen gemäss einem der Ansprüche 1 bis 7, dadurch gekennzeichnet, dass er Indexierungs- und/oder Fortführungsmittel (6, 7) enthält.

**9.** Streifen gemäss Anspruch 8, dadurch gekennzeichnet, dass er auf dem Ende des Trägers, wo keine Membran vorhanden ist, Löcher (6, 7) aufweist.

10. Herstellungsverfahren eines Analyse- order Teststreifens gemäss einem der Ansprüche 1 bis 9, dadurch gekennzeichnet, dass man eine Nitrozellulosefolie (8), oder eine Folie von anderem Material mit gleichen Eigenschaften, auf eine vorerst mit mehreren Löchern durchgebohrte Trägerfolie (9) aus halbsteifem Material anbringt, die Membranfolie (8) dann auf die Trägerfolie (9) fixiert, die Reagenzien auf die einanderfolgenden Flächenstellen niederlegt und die Nitrozellulosemembran mit Protein sättigt.

11. Verfahren gemäss Anspruch 10, dadurch gekennzeichnet, dass aus den Folien (8, 9), die mehrere Streifen liefern, die Gesamtfolien im noch feuchten Zustand nach Sättigung mit protein geschnitten werden.

12. Verfahren gemäss einem der Ansprüche 11 und 12, dadurch gekennzeichnet, dass die Membranfolie (8) an die Trägerfolie (9) durch Schweissen oder Ultraschall gebunden wird.

13. Verfahren gemäss Anspruch 12, dadurch gekennzeichnet, dass das Schweissen durch Erhitzung und Membranentfernung zwischen aufeinanderliegenden reaktiven Flächen realisiert wird, sodass dadurch Zonen (5) ohne Membrane zwischen aufeinanderfolgenden reaktiven Stellen (2) gebildet werden.

14. Verfahren gemäss Anspruch 13, dadurch gekennzeichnet, dass die Nitrozellulosefolie (8) durch eine dünne Schutzfolie abgedeckt wird, und diese Schutzfolie dann aberhoben wird, um die Nitrozellulose vor Erhitzung und Schweissen der Streifen auf dem Träger (1) freizumachen.

15. Verfahren gemäss einem der Ansprüche 10 bis 14, dadurch gekennzeichnet, dass die Membranfolie (8) auf die Trägerfolie (9) geleimt wird.

16. Verfahren gemäss einem der Ansprüche 10 bis 15, dadurch gekennzeichnet, dass die Reagenzien als gerade Linien mittels Stylos oder als Punkte mittels Mikropipetten angebracht werden.

17. Verfahren gemäss Anspruch 11, dadurch gekennzeichnet, dass die Gesamtfolien im feuchtem Zustand nach Imprägnierung der Streifen mit Protein und transversal zu der verbleibenden Folien mittels einer Schneidemaschine geschnitten werden, die mit einer Vorrichtung zum Stapeln der geschnittenen Streifen verbunden ist.

18. Streifen gemäss Anspruch 13, dadurch gekennzeichnet, dass die alternierenden Flächen von Nitrozellulose mittels einer transversalen Reihe von Hitzekörpern, die auf der Membranefolie (9) fortschreiten, erhitzt und verbrannt werden.

Fig.1

Fig.2

Fig.3